(19) Europäisches Patentamt
European Patent Office
Office européen des brevets

(11) Publication number: **0 195 717 B1**

(12) # EUROPEAN PATENT SPECIFICATION

(45) Date of publication of patent specification: **09.10.91**

(51) Int. Cl.5: **C12P 7/40**, C12P 17/14, C12P 41/00

(21) Application number: **86400558.2**

(22) Date of filing: **17.03.86**

(54) **Process for the biotechnological preparation of optically active alpha-arylalkanoic acids.**

(30) Priority: **22.03.85 IT 2003685**

(43) Date of publication of application:
**24.09.86 Bulletin 86/39**

(45) Publication of the grant of the patent:
**09.10.91 Bulletin 91/41**

(84) Designated Contracting States:
**AT BE CH DE FR GB IT LI NL SE**

(56) References cited:

**BIOTECHNOLOGY AND BIOENGINEERING, vol. 26, 1984, pages 1449-1454, John Wiley & Sons, Inc.; B. CAMBOU et al.: "Comparison of different strategies for the lipase-catalyzed preparative resolution of racemic acids and alcohols: asymmetric hydrolysis, esterification, and transesterification"**

**JOURNAL OF THE AMERICAN CHEMICAL SOCIETY, vol. 106, 1984, pages 2687-2692, American Chemical Society; B. CAMBOU et al.: "Preparative production of optically active esters and alcohols using esterase-catalyzed stereospecific transesterification in organic media"**

**AGRICULTURAL & BIOLOGICAL CHEMISTRY, vol. 45, no. 6, June 1981, pages 1389-1392, Tokyo, JP; S. IRIUCHIJIMA et al.: "Asymmetric hydrolysis of (+/-)-alpha-substituted carboxylic acid esters with microorganisms"**

(73) Proprietor: **Montedison S.p.A.**
**31, Foro Buonaparte**
**I-20121 Milan(IT)**

(72) Inventor: **Cesti, Pietro**
**51 Via Torino**
**I-28069 S Martino Di Trecate Novara(IT)**
Inventor: **Piccardi, Paolo**
**8 Via E. De Marchi**
**I-20125 Milan(IT)**

(74) Representative: **Hirsch, Marc-Roger**
**Cabinet Hirsch 34 rue de Bassano**
**F-75008 Paris(FR)**

**EP 0 195 717 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention concerns a process for the biotechnological preparation of arylalkanoic acids resolved in the form of optical S(+) and R(-) isomers.

More particularly, the present invention relates to the biotechnological preparation of α-arylalkanoic acids substantially in the form of an optical S(+) isomer.

Certain α-arylalkanoic acids and, in particular, α-arylpropionic acids used clinically as non-steroidic anti-inflammatory agents are for instance known from T.Y. Shen, Angew Chem., Int. Ed. Engl., 11, 460, 1972,

Since said acids have a centre of a symmetry in position α, they present two optically active S(+) and R(-) enantiomeric forms.

Moreover, it is also known from said article that the anti-inflammatory activity of one of the enantiomers is greater than the activity of the other, as in the case of a large number of members of the class of α-methyl-arylacetic acids known by the trade marks: Ibuprofen®, Naproxen®, Fenoprofen®, etc.

Usually, the S(+) enantiomer develops an activity superior to that of the R(-) enantiomer; this is the case of Ibuprofen® (S.S. Adams et al., in Journal Pharm. Pharmacy 28, 256, 1976) or of Naproxen®, for which the activity of the S(+) enantiomer is about 28 times greater than that of the R(-) enantiomer.

It is likewise known to separate the optical isomers from said acids, see for example D.G. Kaiser et al., in J. Pharm. Science, 2, 269, 1976, and A. Frank and C. Rüchards in Chemistry Letters, 1431-1434, 1984.

However, the known methods for resolving the two optical isomers are complicated, need costly optically active reactants, e.g. the kyral agents such as methylbenzylamine, and do not allow, in many cases, to achieve satisfactory yields, thereby proving unsuitable for use in industry.

Consequently, there was a need for disposing of a method that would allow the resolution of the optical isomers of the above-mentioned acids in a simple and inexpensive manner that would furthermore be efficient and industrially realizable.

Thus, one object of the present invention is to provide a process for the separation of the optical isomers from the α-arylalkanoic acids, in a simple and economical way which is industrially advantageous.

A further object of the present invention is to obtain as efficiently as possible α-arylalkanoic acids substantially in the form of their S(+) optical isomers.

It has now been found by the present Applicant that these and other objects are achieved by a biotechnological process of enzymatic asymmetric hydrolysis of the racemic esters of the α-arylalkanoic acids, capable of supplying an acid rich in S(+) form.

The process of the invention consists in the use of an enzyme produced by a microorganism that is capable of asymmetrically hydrolysing the racemic ester of the above-mentioned acids, i.e. capable of selectively hydrolysing the S(+) enantiomer of the racemic ester, in order to yield an acid substantially in the S(+) form, while leaving the ester in the R(-) form substantially unaltered.

The S(+) acid may easily be separated from the R(-) ester. Thus, a further object of the present invention is to provide a process for the biotechnological separation of α-arylalkanoic acids, substantially in the form of an S(+) optical isomer, consisting in reacting a racemic (R,S) ester of an α-arylalkanoic acid of the formula:

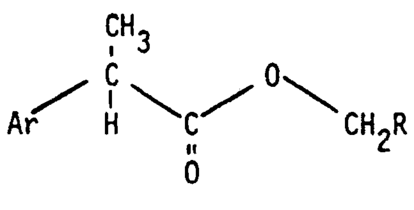

(I)

wherein:

- R represents a -C≡CH, $-CH=CH_2$, -CN, $-COCH_3$, -COO-alk or $CH_2$-O-alk groups, where the said alkyl group comprises 1 to 4 carbon atoms
- Ar represents an aryl or an aryl substituted or condensed with other groups, in particular a group of the formula:

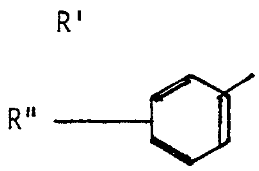 (II) or 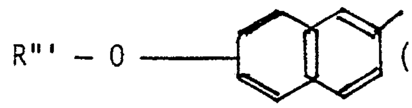 (III)

wherein:

R' is either a linear or a branched alkyl containing from 1 to 8 carbon atoms; an alkenyl containing from 1 to 4 carbon atoms, an alcoxy, a phenyl, a phenoxy, a tenoyl or a heterocyclic radical;

R" is hydrogen or halogen;

R"' is an alkyl radical containing from 1 to 4 carbon atoms, with A CANDIDA CYLINDRACEA, produced by microorganisms capable of asymmetrically hydrolysing, in a predominant way, the S(+) form of the racemic ester of formula (I), and successively in separating with known techniques the acid obtained substantially in the S(+) form from the unreacted ester which is substantially in the R(-) form.

The racemic esters of formula (I) may be prepared according to the conventional esterification processes, for example according to the following reactions:

a) 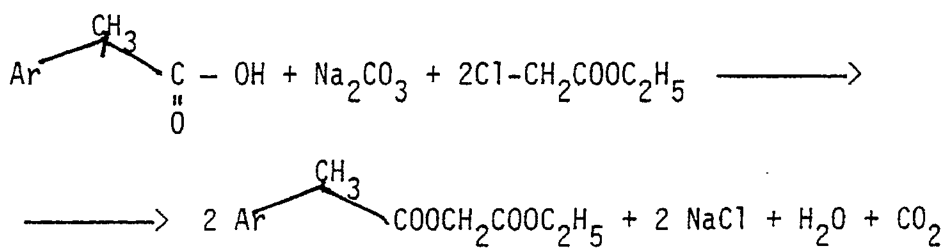

b) 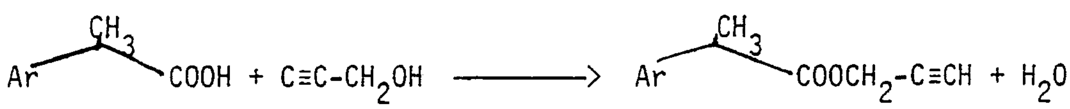

c) 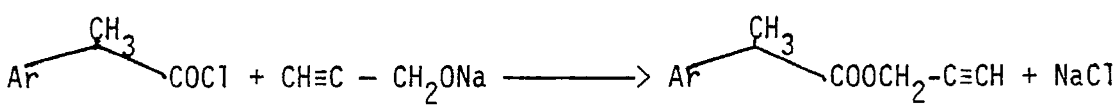

The esterase which may be used according to the process of the present invention is a water-soluble lipase and is produced by microorganisms.

Particularly suitable for the purposes of this invention proved to be the lipase produced by the microorganism "CANDIDA CYLINDRACEA" filed under n° 14830 with the "American Type Culture Collection, USA" (ATCC).

Said lipase is, furthermore, commercially available. The microorganism producing such a lipase may be grown on a liquid nutritional soil according to conventional procedures, for example by inoculating the microorganism into a sterilized liquid culture medium and causing to grow on an alternating shaker at between 20° C and 40° C for a period of 1 to 3 days.

The asymmetric hydrolysis reaction of the racemic esters of formula (I) is carried out by vigorously stirring the mixture of racemic ester with raw or purified esterase which may be contained in a liquid such as water or in the same culture broth of the microorganism, or its extracts or concentrates, or with the suspensions of the microorganism cells.

Alternatively, the enzyme to be used may be supported by a substrate of various nature, chosen according to the techniques of the Prior Art in that specific field.

The process according to the present invention may be conducted at temperatures comprised between 10° and 60° C, but preferably comprised between 20° C and 40° C, while maintaining the pH value of the reaction mixture to values comprised between 5 and 9, but preferably comprised between 6 and 8, values at which the enzymes seem to develop the greatest activity.

The pH of the reaction medium is maintained constant through the use of a buffer solution or by means of the neutralization of the acidity that is formed with a base such, as for example, KOH, NaOH, etc.

The concentration of the starting racemic ester in the reaction mixture may vary from 1% to 20% by weight.

The duration of the asymmetric hydrolysis reaction may vary from 5 to 64 hours, depending upon the specific activity of the enzyme used, which in general depends upon its degree of purity.

At the end of the asymmetric hydrolysis reaction, the acid that has formed and is richly contained in the S(+) isomer on the one part and the ester which is not reacted and is rich in the R(-) isomer on the other part may be extracted from the reaction mixture by using water-non-miscible organic solvents such as, for example, methylene chloride, toluene, lygroine, ethyl ether and analogue solvents.

From the organic extract thus obtained and successively concentrated, the acid, substantially in the S-(+) form may then be separated from the ester which is substantially in the R(-) form, by passing the extract through a chromatographic column, using as a stationary phase, for example, a silica gel.

Alternatively, the rich acid in the S(+) enantiomer may be isolated through extracting it from the reaction mixture by means of basic washups, using for this purpose, for example, aqueous solutions of NaOH at 5% concentration or KOH at a 10% concentration.

The unhydrolised ester which is rich in the R(-) form and has been separated as indicated above, may then be subjected to a racemization by treating it with bases in a anhydrous medium and successively reutilizing it as starting material in the process of this invention, i.e. it may be further subjected to an enzymatic asymmetric hydrolysis treatment.

In a more complicated and, in general, less convenient way, said R(-) ester may be hydrolysed to R(-) acid which, after suitable racemization and subsequent re-esterification steps, will provide a (S,R) racemic ester which may be recycled for use in the process of the invention.

## EXAMPLE 1

### Preparation of S(+) 2-(4-isobutylphenyl)propionic acid

To 100 ml of a buffer solution of 0.2M of $NaHPO_4$ and $KH_2PO_4$ at a pH of 7, were added 2.92 g of ethoxycarbomethyl ester of (R,S) 2-(4-isobutylphenyl)propionic acid, 400 mg of the lipase enzyme produced by the microorganism "CANDIDA CYLINDRACEA" (marketed by SIGMA Chemical Co. USA, with 30% of proteins, and displaying an activity of 1400-2800 units per mg of protein).

The mixture was vigorously stirred for 24 hours, at a temperature of 28° C. The mixture was then extracted with methylene chloride. This extract was analyzed for HPLC (Erbasil RP-18, $CH_3CN$:$H_2O$ 75:25, 254 nm, UV-detection) and the conversion was calculated on the basis of the relationships or ratios between the peaks of the acid that had formed and those of the unreacted ester. The HPLC analyses have proved a conversion rate of 45%, equal to 90% of hydrolysis of the S(+) form. The extract was concentrated and subjected to chromatography on column, using as a stationary phase a silica gel and as an eluent a hexane ether mixture in the ratio of 3:1.

The structure of the acid was confirmed by the NMR analysis. The solid product thus obtained proved to have a melting point equal to 49° C and a rotatory power $[\alpha]_D^{20}$ = +55.55° (C = 1, $C_2H_5OH$).

The GLC analyses, as S(-) $\alpha$-methylbenzamide derivative, have shown an optical purity $\geq$ 95%.

## EXAMPLE 2

To 100 ml of a 0.1M KCl solution were added 2.4 g of propargyl ester of the (R,S) 2-(4-isobutylphenyl)-propionic acid, and 400 mg of the lipase enzyme produced by the microorganism "CANDIDA CYLINDRACEA" of example 1.

The mixture was vigorously stirred maintaining the pH at a constant value of 6.8 with the addition of an aqueous solution of 0.5M KOH at a temperature of 37° C.

After 48 hours, on the basis of the quantity of consumed KOH, it was found that the conversion rate to 2-(4-isobutylphenyl)propionic S(+) acid was equal to about 40%.

The successive separations and analyses were conducted in accordance with the procedures indicated in example 1. The solid product showed a $[\alpha]_D^{20}$ = +54.9° (C = 1, $C_2H_5OH$) and a melting point of 49° C.

## EXAMPLE 3

Into a flask of 500 cc capacity were placed 100 cc of culture soil prepared by dissolving 10 g of

glucose, 5 grams of peptone, 3 g of malt extract and 2 g of yeast extract in 1 litre of $H_2O$, and the pH was then brought up to 6.5.

After sterilization, the culture soil was inoculated with a SLANT of CANDIDA CYLINDRACEA A.T.C.C. n° 14830 microorganism and was then put to incubate for 52 hours at 30° C in a rotary shaker revolving at 350 rpm.

The pH of the reaction medium was then adjusted to value 7, using for this purpose an aqueous solution of 0.5M of KOH, to which were then added 2.92 g of ethoxycarbomethyl ester of the (R,S) 2-(4-isobutylphenyl)propionic acid. This mixture was thereupon subjected to stirring for 48 hours at a temperature of 30° C, while the pH was kept constant by the slow addition of an aqueous solution of 0.5M KOH.

The successive separations and analyses where conducted in accordance with the procedures indicated in example 1.

The S(+) 2-(4-isobutylphenyl)propionic acid, obtained with a conversion rate of 38%, proved that: $[\alpha]_D^{20} = +53.2^\circ$ (C = 1, $C_2H_5OH$) and a melting point of 48° C.

## EXAMPLE 4

### Preparation of 2-(6-methoxy-2-naphthyl)propionic acid

To 100 ml of a 0.3M buffer solution of phosphate ($NaHPO_4$ + $KH_2PO_4$) were added 3.2 g of cyanomethyl ester of 2-(6-methoxy-2-naphthyl)propionic acid, and 500 mg of the CANDIDA CYLINDRACEA enzyme of example 1. This mixture was then vigorously stirred for 52 hours at a temperature of 32° C. The analyses showed a conversion rate of the (R,S) racemic ester to the corresponding S(+) acid equal to 40%.

The separations and analyses were conducted in accordance with the procedures followed in example 1.

The product thus obtained was crystallized from an acetone/hexane mixture and found to have the following characteristics: melting point (m.p.) = 154° C; $[\alpha]_D^{20} = +65.14^\circ$ (C = 1, $CHCl_3$).

## EXAMPLES FROM 5 TO 10

Operating was carried out according to the procedures described in example 1 by using buffer solutions of $NaHPO_4$ and $KH_2PO_4$ with a pH = 6.5, at a temperature of 32° C and with the reactants indicated on table I.

The conversion rates corresponding to the S(+) acids as indicated in table I were obtained, said conversion rates being calculated either with respect to the starting (R,S) racemic ester or with respect to the S(+) ester. The optical purities of the acids thus obtained proved to be consistently greater than 90%.

TABLE I

| Ex. | Racemic ester (R,S) $Ar-\overset{CH_3}{\overset{\vert}{C}H}-COOCH_2R$ | | Racemic ester (R,S) | Lipase enzyme from Candida Cylindracea | Buffer ($NaHPO_4$ + $KH_2PO_4$) | Reaction time | Conversion racemic ester (R,S) | Conversion S(+)ester |
|---|---|---|---|---|---|---|---|---|
| | Ar | R | g | g | ml | hrs | % | % |
| 5 | 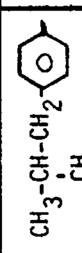 | $-CH=CH_2$ | 2 | 0.3 | 50 | 45 | 48 | 96 |
| 6 | 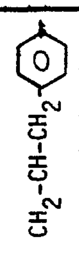 | $-\overset{\overset{O}{\Vert}}{C}-CH_3$ | 3 | 0.2 | 100 | 30 | 35 | 68 |
| 7 | 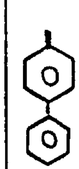 | -CN | 3 | 0.4 | 70 | 42 | 40 | 76 |
| 8 | 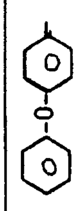 | $-COOCH_3$ | 4 | 0.4 | 100 | 28 | 30 | 59 |
| 9 | 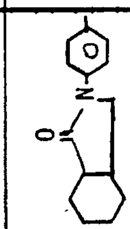 | -CN | 2 | 0.2 | 50 | 24 | 47 | 92 |
| 10 | 6-methoxy-2-naphthyl | $CH_2OC_2H_5$ | 3 | 0,5 | 70 | 32 | 32 | 60 |

## Claims

1. Process for the biotechnological preparation of α-arylalkanoic acids, substantially in the form of an optical S(+) isomer, said process consisting in reacting a racemic (R,S) ester of an α-arylalkanoic acid

of the formula:

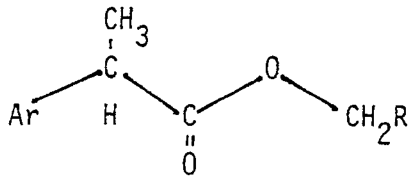

(I)

wherein:

R represents a -C≡CH, $-CH=CH_2$, -CN, $-COCH_3$, -COO-Alk or $-CH_2$-O-alk group where the alkyl group contains 1 to 4 carbon atoms,

Ar represents a group of the formula:

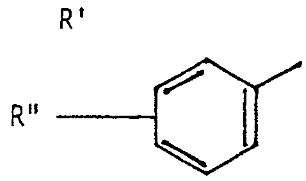

(II) or

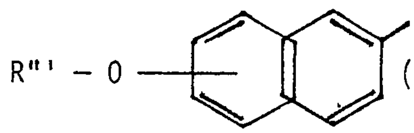

(III)

wherein:

R' is either a linear or a branched alkyl containing from 1 to 8 carbon atoms; an alkenyl containing from 1 to 4 carbon atoms, an alcoxy, a phenyl, a phenoxy, a tenoyl, a heterocycle.

R" is hydrogen or halogen;

R"' is an alkyl containing from 1 to 4 carbon atoms

with a CANDIDA CYLINDRACEA lipase produced by microorganisms capable of asymmetrically hydrolysing, in a predominant way, the S(+) form of the racemic ester of formula (I) and in separating, using known techniques, the acid obtained, substantially in the S(+) form, from the unreacted ester which is substantially in the R(-) form.

2. Process according to claim 1, characterized in that the said lipase is oontained in the cells of the microorganisms or in a culture liquid of the same or in extracts or concentrates of the same.

3. Process according to claim 1 or 2, characterized in that the asymmetric hydrolysis reaction is conducted at a pH comprised between 5 and 9.

4. Process according to any one of claims 1 to 3, characterized in that the reaction temperature is comprised between 20° C and 40° C.

5. Process according to any one of claims 1 to 4, characterized in that the concentration of the racemic ester of formula (I) in the reaction mixture is comprised between 1% by weight and 20% by weight.

**Revendications**

1. Procédé de préparation biotechnologique d'acides α-arylalkanoïques, sensiblement sous forme d'un isomère optique S(+), ledit procédé consistant à faire réagir un ester racémique (R,S) d'un acide α-arylalcanoïque de formule:

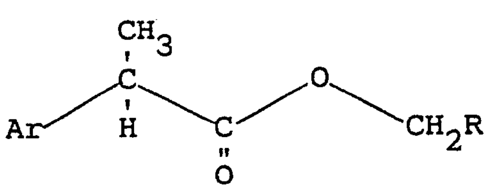

(I)

dans laquelle:

R représente un groupement -C≡CH, -CH-CH$_2$, -CN, -COCH$_3$-, -COO-Alk ou -CH$_2$-O-alk dans lequel le groupe alkyle contient de 1 à 4 atomes de carbone;

Ar représente un groupe de formule:

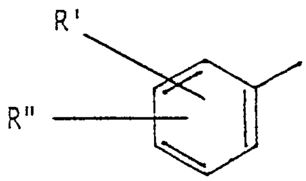
(II) or

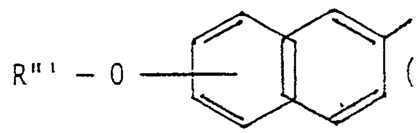
(III)

dans laquelle:

R' est soit un groupe alkyle linéaire, soit un groupe alkyle branché contenant de 1 à 8 atomes de carbone; un groupe alkényle contenant de 1 à 4 atomes de carbone, un groupe alkoxy, phényl, phénoxy, phénol, ou un hétérocycle;

R" est un atome d'hydrogène ou un halogène;

R''' est un groupe alkyle contenant de 1 à 4 atomes de carbone;

avec une lipase CANDIDA CYLINDRACEA produite par des microorganismes susceptibles d'hydrolyser asymétriquement, selon une direction prédominante, la forme S(+) de l'ester racémique de formule (I) et de séparer, par des techniques connues, l'acide obtenu, sensiblement sous la forme S(+), de l'ester n'ayant pas réagi qui se présente sensiblement sous la forme R(-).

**2.** Procédé selon la revendication 1, caractérisé en ce que ladite lipase est contenue dans les cellules de microorganismes ou dans un de leurs milieux de culture ou extraits ou concentrés de ces cultures.

**3.** Procédé selon la revendication 1 ou 2, caractérisé en ce que la réaction d'hydrolyse asymétrique est mise en oeuvre à un pH compris entre 5 et 9.

**4.** Procédé selon l'une quelconque des revendications 1 à 3, caractérisé en ce que la température réactionnelle est comprise entre 20 et 40 °C.

**5.** Procédé selon l'une quelconque des revendications 1 à 4, caractérisé en ce que la concentration de l'ester racémique de formule (I) dans le mélange réactionnel est comprise entre 1 et 20% en poids.

**Patentansprüche**

**1.** Verfahren zur biotechnologischen Herstellung von alpha-Arylalkansäuren, im Wesentlichen in Form eines optischen Isomers S(+), das darin besteht das racemische Ester (R,S) einer Alpha-Arylalkansäure der Formel:

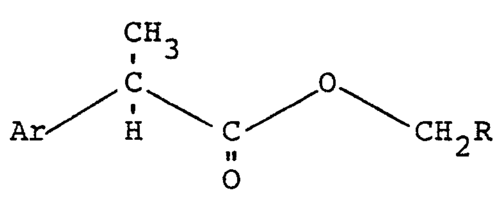
(I)

in welcher:

R eine Gruppierung -C=CH, -CH-CH$_2$, -CN, -COCH$_3$-, -COO-Alk oder -CH$_2$-O-alk darstellt, in welcher die Alkylgruppe 1 bis 4 Kohlenstoftatome enthält;

Ar zeigt eine Formelgruppe:

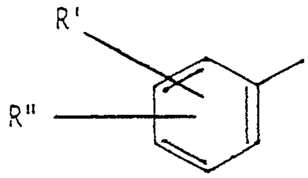 (II) or 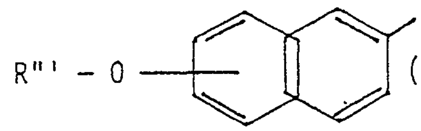 (III)

in der:

R' entweder eine lineare oder verzweigte Alkylgruppe, mit 1 bis 8 Kohlenstoffatomen; eine Alkenylgruppe mit 1 bis 4 Kohlenstoffen, eine Gruppe Alkoxy, Phenyl, Phenoxy, Phenol, oder eine heterocyclische Verbindung;

R'' ein Wasserstoffatom oder ein Halogenatom;

R''' eine Alkylgruppe mit 1 bis 4 Kohlenstoffen ist;

reagieren zu lassen mit einer CANDIDA CYLINDRACEA Lipase welche durch Mikroorganismen produziert ist die fähig sind, in einer dominierenden Richtung, die Form S(+) des racemischen Esters der Formel (I) asymmetrisch zu hydrolysieren und die gewonnene Säure, im Wesentlichen in Form S(+), durch bekannte Techniken von dem unreagierten Ester, das im Wesentlichen in Form R(-) erscheint, zu trennen.

**2.** Verfahren nach Anspruch 1, dadurch gekennzeichnet, dass die Lipase in den Zellen der Mikroorganismen, in einer flüssigen Kultur, in Extrakten oder in Konzentraten der selben enthalten ist.

**3.** Verfahren nach Anspruch 1 oder 2, dadurch gekennzeichnet, dass die Reaktion der asymmetrischen Hydrolyse mit einen pH zwischen 5 und 9 durchgeführt wird.

**4.** Verfahren nach einem der Ansprüche 2 bis 3, dadurch gekennzeichnet, dass die Reaktionstemperatur zwischen 20 und 40°C liegt.

**5.** Verfahren nach einem der Ansprüche 1 bis 4, dadurch gekennzeichnet, dass die Konzentration des racemischen Esters der Formel (I) in der Reaktionsmischung zwischen 1 und 20 Gewichtsprozent beträgt.